Europäisches Patentamt

European Patent Office

Office européen des brevets

(11) **EP 1 527 785 A1**

(19)

(12) # EUROPEAN PATENT APPLICATION

(43) Date of publication:
**04.05.2005 Bulletin 2005/18**

(51) Int Cl.[7]: **A61K 38/19**, A61P 9/10,
A61P 25/00, A61P 13/12,
A61P 1/16

(21) Application number: **03024526.0**

(22) Date of filing: **27.10.2003**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(71) Applicant: **Ludwig-Maximilians-Universität
München
80539 München (DE)**

(72) Inventors:
• **Franz, Wolfgang-Michael
82234 Wessling (DE)**
• **Engelmann, Markus Georg
82222 Eichenau (DE)**
• **Steinbeck, Gerhard
82340 Feldafing (DE)**

(74) Representative: **VOSSIUS & PARTNER
Siebertstrasse 4
81675 München (DE)**

(54) **Use of G-CSF for treating ischemia**

(57)    The present invention relates to uses of granulocyte colony stimulating factor (G-CSF) or fragment thereof for the preparation of a pharmaceutical composition for treating organ dysfunction caused by ischemia, whereby the pharmaceutical composition is to be administered to a patient who is subjected to a surgical or interventional procedure in order to improve organ function, to improve organ function, to improve blood flow and/or to induce revascularization.

Furthermore, the present invention relates to methods of treating organ dysfunction caused by ischemia comprising administering a therapeutically effective amount of G-CSF or fragment thereof to a patient who is subjected to a surgical or interventional procedure in order to improve organ function, to improve organ function, to improve blood flow and/or to induce revasculization.

EP 1 527 785 A1

## Description

The present invention relates to uses of granulocyte colony stimulating factor (G-CSF) or fragment thereof for the preparation of a pharmaceutical composition for treating organ dysfunction caused by ischemia, whereby the pharmaceutical composition is to be administered to a patient who is subjected to a surgical or interventional procedure in order to improve organ function, function, to improve blood flow and/or to induce revascularization.

Furthermore, the present invention relates to methods of treating organ dysfunction caused by ischemia comprising administering a therapeutically effective amount of G-CSF or fragment thereof to a patient who is subjected to a surgical or interventional procedure in order to improve organ function, function, to improve blood flow and/or to induce revasculization.

Cardiovascular disease is the number one cause of mortality in Western countries and claims approximately 1 million lives each year in the United States. The majority of cardiovascular deaths are due to coronary artery disease (CAD). CAD is a progressive disease that results in a spectrum of clinical manifestations, ranging from asymptomatic atherosclerosis and stable angina to the acute coronary syndromes (i.e., unstable angina, myocardial infarction (MI), and sudden ischemic death). The treatment of choice for acute ST segment elevation MI is either thrombolysis or primary coronary intervention (PCI) when coronary catheterization is immediately accessible. These revascularization strategies are well established in treatment of acute MI when treatment is initiated very early after onset of infarction (usually within 6 hours). The mortality benefit of thrombolytic therapy for acute MI is strongly dependent on time to treatment (Cannon (2000) *Jama.* 283: 2941-2947). Primary PCI is an acceptable alternative to thrombolytic therapy in patients with acute MI and may result in superior outcomes in selected patient populations, especially the elderly, patients with prior coronary artery bypass surgery, those with congestive heart failure, and those in cardiogenic shock. Clinical trials support the use of primary PCI as first-line therapy for acute MI. Patients in whom thrombolytic therapy is contraindicated or known to have reduced efficacy are also excellent candidates for this therapy (Degeare (2001) *Am Heart J.* 141:15-24). The use of coronary stents and glycoprotein IIb/IIIa antagonists resulted in complementary long-term clinical benefits in terms of decreased morbidity and incidence of death or myocardial infarction (Lincoff (1999) *N Engl J Med.* 341:319-327). Early administration of glycoprotein IIb/IIIa antagonists in patients with acute myocardial infarction improves coronary patency before stenting, the success rate of the stenting procedure, the rate of coronary patency at six months, left ventricular function, and clinical outcomes (Montalescot (2001) *N Engl J Med.* 344:1895-1903). However, the treatment of patients who are admitted at a later stage to the hospital is still controversial, since thrombolysis failed to improve mortality and morbidity in these patients. Recent observations suggest that time to treatment may be less important with primary percutaneous transluminal coronary angioplasty (PTCA), but re-infarction and infarct artery re-occlusion at 6 months were shown to be more frequent with later reperfusion using PTCA (>6 hours) (Brodie (2001) *Am J Cardiol.* 88: 1085-1090). Late coronary stenting of the infarct-related artery in patients with acute myocardial infarction is a safe and effective late reperfusion therapy and may be beneficial to the patients (Hsieh (1998) *Am Heart J.* 136: 606-612). Since patients who are admitted to the emergency room very late after onset of acute MI are threatened mainly by left ventricular remodeling that leads to sudden cardiac death or progressive heart failure, these patients may benefit from therapeutic strategies focussing on supporting myocardial function via improved neoangiogenesis and/or tissue regeneration. Although neoangiogenesis within the infarcted tissue is an integral component of the remodeling process, the capillary network is unable to support the greater demands of the hypertrophied myocardium, resulting in progressive loss of viable tissue, infarct extension and fibrous replacement. Bone marrow from adult humans contains stem cells and precursor cells with phenotypic and functional characteristics of embryonic hemangioblasts, and these can be used to directly induce new blood vessel formation in the infarct-bed (vasculogenesis) and proliferation of preexisting vasculature (angiogenesis) after experimental myocardial infarction.

The neoangiogenesis was shown to decrease apoptosis of hypertrophied myocytes in the peri-infarct region, long-term salvage and survival of viable myocardium, reduction in collagen deposition and sustained improvement in cardiac function in a mouse model of myocardial infarction (Kocher (2001) *Nat Med.* 7:430-436). Direct injection of bone marrow cells in a porcine model of myocardial infarction resulted in improvement of myocardial function via induction of myogenesis and angiogenesis (Tomita (2002) *J Thorac Cardiovasc Surg.*123: 1132-1140). Early uncontrolled human clinical studies investigating the effect of autologous bone marrow stem cell transplantation into the acutely infarcted heart were shown to be safe and demonstrated some improvement of regional left ventricular function (Strauer (2002) *Circulation.* 106:1913-1918; Assmus (2002) *Circulation.* 106:3009-3017; Stamm *(2003) Lancet* 361:45-46).

Another option of stem cell therapy is the endogenous mobilization of stem cells via cytokines such as G-CSF or GM-CSF. The myeloid growth factors granulocyte-colony stimulating factor (G-CSF) and granulocyte-macrophage-colony stimulating factor (GM-CSF) have been shown to stimulate production of neutrophils in various clinical settings.

In cancer patients undergoing intensive cytotoxic chemotherapy, G-CSF stimulation of granulopoiesis translated into decreased incidence of neutropenia-associated fever and less infection in patients with a likelihood of developing granulocytopenia (Dempke (2000) *Anticancer Res*.20:5155-5164). G-CSF has also been studied in patients with AIDS, congenital and cyclic neutropenia, myelodysplastic syndromes, and aplastic anemia; it increases the neutrophils count in many patients with these disorders (Anderlini (1997) *Blood.* 90:903-908). In neutropenic patients, G-CSF often increases the neutrophils count enough to decrease the frequency and severity of infection. G-CSF has been well tolerated, with no apparent dose-limiting toxicity (Pettila, (2000), Crit. Care Med. 28: 3620-3625). Administration in normal subjects, an increase in absolute neutrophil count by 6- to 8-fold and a CD34+ cell count more than 30-fold at the highest doses tested (10 µg/kg/d) were observed for at least 1 week. Mobilization of stem cells is thought to be restricted to the myeloid lineage, though in other species other stem cell populations were characterized (Falanga (1999) *Blood.* 93:2506-2514; Natori (2002) *Biochem Biophys Res Commun.* 297:1058-1061; Gehling (2000) *Blood.* 95:3106-3112; Yang (1998) *Blood*. 92: 4632-4640). Characterization of released stem cells after G-CSF treatment in humans suffering from ischemic syndromes are not known.

Recently, repair of infarcted myocardial tissue was demonstrated resulting in improved ventricular function and survival in mice after bone marrow cell mobilization induced by granulocyte-colony stimulating factor (G-CSF) and stem cell factor (SCF), when both cytokines were administered before establishment of infarction (Orlic (2001) *Proc Natl Acad Sci U S A*. 98: 10344-10349). A similar administration protocol of G-CSF was used in a baboon model showing ventricular function was not improved when given prior to experimental infarction (Norol (2003) *Blood.* 28:28). G-CSF was used in a rat model to treat cerebral ischemia and resulted in a decrease of mortality as well as reduction of cerebral infarct area (Six (2003) *Eur J Pharmacol*. 458:327-328).

Non-cardiac adverse events during G-CSF treatment are usually not serious (van Der Auwera (2001) Am. J. Hematol. 66:245-251). In most clinical trials observed adverse effects contain discrete to moderate (10%), in some cases severe (3%) bone and muscle pain which can usually be treated using standard analgesics. More common transient elevation of serum urea, LDH, alkaline phosphatase or liver function tests occur. In rare cases lowered blood pressure is observed (Fachinformation Neupogen, 2002 AMGEN). Since patients suffering from ischemic syndromes such as myocardial infarction are mainly threatened by occlusion of the infarct vessel by thrombus formation onto ruptured atheromatous plaques, major concerns against administration of G-CSF in those populations include impairment of arterial flow caused by high amounts of leukocytes and neutrophil activation resulting in acceleration of reperfusion injury (Kilgore (2000) *Am Heart J.* 139:32-34). Increased expression of neutrophil and monocyte adhesion molecules may contribute to their adhesion to endothelium in the ischemic territory. This adhesion could feasibly precipitate vasoconstriction or add a local thrombotic effect due to tissue factor expression secondary to Mac-1 engagement. In addition, interaction of leukocytes with monocytes expressing ICAM-1 suggests that leukocytes may form microaggregates that could cause microvascular plugging. This mechanism may facilitate the occurrence of the "no-reflow" phenomenon or slow coronary filling after acute myocardial infarction (Meisel (1998) *J Am Coll Cardiol.* 31:120-125). However, a clinical trial investigating patients suffering from stable CAD with chest pain was launched July, 2002. However, patients with acute ischemic syndromes such as myocardial infarction or unstable angina are excluded from this trial (protocol NHLBI 02-H-0264).

In addition, ischemic dysfunction of other organs, such as brain, peripheral extremities, liver, kidney, retina, or spinal cord are major causes of either morbidity and mortality in humans. Cerebro-vascular diseases (CVD), in particular, cause approximately 200.000 deaths in the United States each year as well as considerable neurological disability. Ischemia and infarction constitute 85 to 90 percent of CVD, while 10 to 15 percent are intercranial hemorrhages. Though established therapies of prevention such as surgical or angioplasty can reduce morbidity and mortality, treatment of acute ischemic brain injury is still unsatisfying and prognosis worsens with the extent of infarction (Easton (1998) in Harrison's principles of internal medicine p2325-2347). Stem cell mobilization might be an alternative approach by using G-CSF resulted in reduction of brain infarct volume in mice and might have a therapeutic potential (Six (2003) Eur. J. Pharmacol. 458: 327-328).

As in patients with atherosclerosis of the coronary and cerebral vasculature, peripheral occlusive arterial disease (PAD) is seen most frequently in the 6th and 7th decades of life. Severe presentation of PAD is critical limb ischemia which is characterized by persistent ischemic pain at rest. Prognosis of those patients is poor, since the 5 year mortality is up to 60%, and most patients die from myocardial infarction or sudden death (Wolfe (1997) Eur. J. Vase. Endovasc. Surg. 13: 578-582). Administration of endothelial progenitor cells (EPC) was shown to have potential of improving limb perfusion in animal models of ischemia (Takahashi (1999) Nat. Med. 5:434-438).

Acute renal failure is a syndrome characterized by rapid decline of renal function and complicates approximately 5 percent of hospital admissions and up to 30 percent of hospital admissions. Acute ischemia of renal tissue, is one of the major causes of intrinsic acute renal failure and is associated with major in-hospital morbidity and mortality (Brady (1996) in The Kidney 5th ed. Saunders p1200-52). Until now, in most cases current therapy approaches are limited to supportive strategies with a significant risk of permanent loss of organ function. Spinal cord injury, caused either by ischemia or trauma often results in permanent disability and therapeutic options are limited. Retinal ischemia either caused by thrombotic or embolic occlusion of retinal artery or thrombosis of retinal vein usually results in severe impairment of visual function of patients.

Moreover, there is so far no effective treatment of defects caused by ischemic disease in particular for the myocardicum or brain. Another drawback in the art is that the costs for such a treatment, which can even be a heart transplantation in the case of patients with severe ischemic organ damage (e.g. ischemic heart failure after an heart attack), are enormous and present a severe strain on the health system in Western countries. Treatment of brain ischemia or infarction is at present carried out predominantly symptomatically. Therefore, a treatment leading to a better healing of tissue affected by, e.g., an infarct, trauma or stroke would be of great clinical benefit.

In view of the above-described drawbacks and the inefficacy of the currently applied methods for treating ischemia with respect to the resulting organ defects, there is a need for an effective means and methods to provide effective treatment of organ dysfunction caused by ischemic disease. In particular, there is a need for economical and/or clinical efficient methods for the treatment of ischemia.

Thus, the technical problem of the present invention is to comply with the needs described above. The solution to this technical problem is achieved by providing the embodiments characterized in the claims.

Accordingly, in one aspect the present invention relates to the use of G-CSF or fragment thereof for the preparation of a pharmaceutical composition for the treatment of organ dysfunction caused by ischemia, whereby the pharmaceutical composition is to be administered to a patient who is subjected to a surgical or interventional procedure in order to improve organ function, to improve blood flow and to induce revascularization.

The present invention is based on the surprising finding that organ function significantly improves, when G-CSF or fragment thereof is administered to a patient suffering from organ dysfunction caused by ischemia and who is subjected to a surgical or interventional procedure to improve organ function, to improve blood flow and/or to induce revascularisation which is demonstrated in the appended Examples. This finding was obtained from a phase I patient study which is also illustrated in the appended Examples and Figures infra.

In this application a Phase I, randomized, open-label efficacy and safety study compared the effects of peripheral blood stem cells (PBSC) induced by G-CSF on the improvement in various vital cardiac function parameters in ischemic heart failure patients undergoing delayed percutaneously coronary intervention (PCI) for ST segment elevation myocardial infarction (STEMI). The primary objective of this study was to compare between a treatment of G-CSF over, for example, 5 days, first administered, for example, 24 hours after successful PCI, versus Placebo s.c. in terms of myocardial thickness, regional myocardial function and global cardiac function from baseline over 3 months of follow-up. Analysis of cardiac function consisted of evaluation of end-diastolic myocardial thickness, segmental systolic wall thickening, end-diastolic volume, end-systolic volume, stroke volume, ejection fraction and cardiac output by means of magnetic resonance imaging (MRI) and transthoracic echocardiography. The extent of non-viable myocardium was assessed using contrast enhanced MRI. Secondary objectives of this study were the determination of the occurrence of major adverse cardiac events (death, myocardial infarction, coronary bypass grafting, or re-intervention), to evaluate the safety of G-CSF treatment in patients suffering from STEMI.

Until now, 18 patients are included in the study, 6 of them are randomized to receive either G-CSF or placebo. Of initial non-randomized patients (n=4), CD34+ cells increased from $3 \pm 1/\mu L$ (baseline) to $60 \pm 49/\mu L$ at day 5 (p<0.001). No major adverse events were observed in all treated patients. Besides a significant increase in hematopoetic stem cells (CD 34+), it was surprisingly found that other stem cell populations, such as endothelial progenitor cells (EPC), multipotent adult progenitor cells (MAPC), side population (SP) cells, and c-kit+ lineage-negative cells are increased in the peripheral blood. Analyses of MR data of 8 patients who completed the follow-up demonstrate that ventricular volumes and global ventricular function remain unchanged in both placebo and G-CSF treated patients from baseline to 3 months. The number of improved myocardial segments of the infarct region increased from $1 \pm 1$ (placebo) to $2.8 \pm 0.9$ (G-CSF) at 3 months (p=0.085). Regional ventricular thickening of the main infarct segment increased significantly from $0.33 \pm 0.47$ mm (placebo) to $3.3 \pm 0.64$ mm (G-CSF) at 3 months (p=0.019). In conclusion, G-CSF resulted in significant mobilization of hematopoetic and other stem cell populations that were shown to have a regenerative potential on the heart which is demonstrated in the appended Examples, infra. The treatment with G-CSF was safe in STEMI patients undergoing PCI, and regional left ventricular function was significantly improved in the long term.

Thus, the above described study shows that G-CSF has the potential of supporting myocardial regeneration of infarcted tissue after late revascularisation when administered to a patient suffering from organ dysfunction caused by ischemia.

In addition, as is shown in the appended Examples, animal experiments in mice (n=14) were performed to test survival after experimentally induced myocardial infarction. Four mice received G-CSF via the intraperitoneal route after myocardial infarction. Control animals (n=10) were sham operated. Strikingly, it was observed that due to this measure mortality in mice treated with G-CSF was reduced after myocardial infarction. In particular, survival of mice was significantly improved after administration of G-CSF (100% survival vs. 60% survival in sham operated mice).

Accordingly, the present invention provides for the first time a means and methods for the treatment of organ dysfunction caused by ischemia with G-CSF or fragments thereof which reduces non viable organ tissue and is suitable for the combined application with common surgical and interventional measures.

Hematopoietic progenitor cell production is regulated by combinations of cytokines, the so called hematopoietic growth factors. Granulocyte precursor cells give rise to neutrophil granulocyte which is mainly promoted by G-CSF. Furthermore, hematopoietic growth factors orchestrate the body response to infection and other stresses. In response to bacteria G-CSF, inter alia, is produced and secreted into the circulation. It is well known that G-CSF is a type of growth factors that stimulates, inter alia, the bone marrow to make the different types of blood cells. G-SCF is also known to attract and/ or mobilize stem cells from, e.g., the bone marrow or other places within the body on which said stem cells may reside. G-CSF has a molecular weight of approximately 19 kDa and probably forms a dimer. It is a member of the four α-helical cytokine family, and its receptor contains preferably the WSXWS motif.

The term "G-CSF" (granulocyte colony stimulating factor) as used herein relates to a G-CSF polypeptide/polypeptides which is/are well known in the art; e. g., as G-CSF; trade name: Neupogen® (Filgrastim, commercially available from Amgen) or Granocyte® (Lenograstim, commercially available from Chugai Pharma). The amino acid sequence of G-CSF is described, e.g., in Nagata (1986) Nature 319:415-418. It is known that G-CSF has 174 amino acid residues, however, variants thereof are known and are also encompassed by the term "G-CSF" as described infra. For example, a G-CSF polypeptide having an additional methionine residue at its N-terminus is known. The function of G-CSF can be tested by methods known in the art which are described herein.

Said term also encompasses G-CSF polypeptides from preparations well known in the art, either from natural sources or preferably produced by recombinant means. Multiple forms of natural or recombinant human, mouse or rat G-CSF are known in the art. It is envisaged that the G-CSF or fragment thereof used in the context of the present invention is of pharmaceutical grade suitable for administration to patients as described infra.

Said term also encompasses G-CSF polypeptides comprising an amino acid sequence at least 70%, 80%, 90%, 95%, 97% or 99% identical to the G-CSF polypeptide which is known in the art and has preferably G-CSF activity as described supra.

The person skilled in the art is readily in a position to determine the sequence identity of a polypeptide of the G-CSF polypeptide. For example, BLAST2.0, which stands for Basic Local Alignment Search Tool (Altschul, Nucl. Acids Res. 25 (1997), 3389-3402; Altschul, J. Mol.

Evol. 36 (1993), 290-300; Altschul, J. Mol. Biol. 215 (1990), 403-410), can be used to search for local sequence alignments. BLAST produces alignments of both nucleotide and amino acid sequences to determine sequence similarity. Because of the local nature of the alignments, BLAST is especially useful in determining exact matches or in identifying similar sequences. The fundamental unit of BLAST algorithm output is the High-scoring Segment Pair (HSP). An HSP consists of two sequence fragments of arbitrary but equal lengths whose alignment is locally maximal and for which the alignment score meets or exceeds a threshold or cutoff score set by the user. The BLAST approach is to look for HSPs between a query sequence and a database sequence, to evaluate the statistical significance of any matches found, and to report only those matches which satisfy the user-selected threshold of significance. The parameter E establishes the statistically significant threshold for reporting database sequence matches. E is interpreted as the upper bound of the expected frequency of chance occurrence of an HSP (or set of HSPs) within the context of the entire database search. Any database sequence whose match satisfies E is reported in the program output.

Analogous computer techniques using BLAST (Altschul (1997), loc. cit.; Altschul (1993), loc. cit.; Altschul (1990), loc. cit.) are used to search for identical or related molecules in nucleotide databases such as GenBank or EMBL. This analysis is much faster than multiple membrane-based hybridizations. In addition, the sensitivity of the computer search can be modified to determine whether any particular match is categorized as exact or similar. The basis of the search is the product score which is defined as:

$$\frac{\%\text{sequence identity x \% maximum BLAST score}}{100}$$

and it takes into account both the degree of similarity between two sequences and the length of the sequence match. For example, with a product score of 40, the match will be exact within a 1-2% error; and at 70, the match will be exact. Similar molecules are usually identified by selecting those which show product scores between 15 and 40, although lower scores may identify related molecules.

Yet, G-CSF polypeptides of the present invention also encompass variants thereof as is described infra.

The basic structure of polypeptides and the recombinant or synthetic production as well as isolation methods of polypeptides are well known and have been described in innumerable textbooks and other publications in the art. In this context, the term polypeptide is used herein to refer to any peptide or protein comprising two or more amino acid joined to each other in a linear chain by peptide bonds. As used herein, the term refers to both short chains, which also commonly are referred to in the art as peptides, oligopeptides and oligomers, for example, and to longer chains, which generally are referred to in the art as proteins, of which there are may types. It will be appreciated that polypeptides often contain amino acids other than the 20 amino acids commonly referred to as the 20 naturally occurring amino acids, and that many amino acids, including the terminal amino acids, may be modified in a given polypeptide, either by natural processes, such as processing and other post-translational modifications, but also by chemical modification techniques which are well known to the art. Even the common modification that occur naturally in polypeptides are too numerous to list exhaustively here, but they are well described in basic texts and in more detailed monographs, as well as in a voluminous research literature, and they are well known to those of skill in the art.

Among the known modifications which may be present in G-CSF polypeptides for use in the present invention are, to name an illustrative few, acetylatioin, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-linkings, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfurylation, transfer-RNA mediated addition of amino acids to proteins such as arginylation, and ubiquitination.

Such modifications are well known to those of skill and have been described in great detail in the scientific literature. Several particularly common modifications, glycosylation, lipid attachment, sufation, gamma-carboxylation of glutamic acid residues, hydroxylation and ADP-ribosylation, for instance, are described in most basic texts, such as, for instance *PROTEINS - STRUCTURE AND MOLECULAR PROPERTIES,* 2nd ed., T. E. Creighton, W. H. Freeman and Company, New York (1993). Many detailed reviews are available on this subject, such as, for example, those provided by Wold, F., Posttranslational Protein Modifications: Perspectives and Prospects, pgs. 1 to 12 in *POSTTRANSLATIONAL COVALENT MODIFICATION OF PROTEINS,* B. C. Johnson, ed., Academic Press, New York (1983); Seifert et al., *Meth. Enzymol.* 182:626-646 (1990) and Rattan et al., *Protein Synthesis: Posttranslational Modifications and Aging,* Ann. N.Y. Acad. Sci. 663: 48-62 (1992). It will be appreciated, as is well known and as noted above, that polypeptides are not always entirely linear. For instance, polypeptides may be generally as a result of posttranslational events, including natural processing event and events brought about by human manipulation which do not occur naturally. Circular, branched and branched circular polypeptides may be synthesized by non-translational natural process and by entirely synthetic methods, as well. Modifications can occur anywhere in polypeptides, including the peptide backbone, the amino acid side-chains and the amino or carboxyl termini. In fact, blockage of the amino or carboxyl group in a polypeptide, or both, by a covalent modification, is common in naturally occurring and synthetic polypeptides and such modifications may be present in polypeptides of the present invention, as well. For instance, the amino terminal residue of polypeptides made in *E. coli* or other cells as defined above, prior to proteolytic processing, almost invariably will be N-formylmethionine. During post-translational modification of the peptide, a methionine residue at the NH2-terminus may be deleted. Accordingly, this invention contemplates the use of both the methionine-containing and the methionineless amino terminal variants of the protein of the invention. The modifications that occur in a polypeptide often will be a function of how it is made. For polypeptides made by expressing a cloned gene in a host, for instance, the nature and extend of the modifications in large part will be determined by the host cell posttranslational modification capacity and the modification signals present in the polypeptide amino acid sequence. For instance, as is well known, glycosylation often does not occur in bacterial hosts such as, for example, *E. coli.* Accordingly, when glycosylation is desired, a polypeptide should be expressed in a glycosylating host, generally a eukaryotic cell. Insect cell often carry out the same posttranslational glycosyltions as mammalian cells and, for this reason, insect cell expression systems have been developed to express efficiently mammalian proteins having native patters of glycosylation, inter alia. Similar considerations apply to other modifications. It will be appreciated that the same type of modification may be present in the same or varying degree at several sites in a given polypeptide. Also, a given polypeptide may contain many types of modifications. In general, as used herein, the term polypeptide encompasses all such modifications, particularly those that are present in polypeptides synthesized recombinantly by expressing a polynucleotide in a host cell.

Variant(s) of G-CSF polypeptides, as the term is used herein, are polypeptides that differ from a reference polypeptide, respectively. Generally, differences are limited so that the sequences of the reference and the variant are closely similar overall and, in may regions, identical. A variant and reference polypeptide may differ in amino acid sequence by one or more substitutions, additions, deletions, fusions and truncations, which may be present in any combination. Preferably, said variant (s) has/have G-CSF activity as described supra.

The term "fragment thereof" when used in the context of the present invention means fragments of G-CSF polypeptides having G-CSF activity. The amino acid sequence of G-CSF and of corresponding variants is known in the art and published in Nagata (1986), Nature 319:415-418. Accordingly, said G-GSF fragments comprise portions of 5, 10, 15, 20, 25, 30, 35, 40, 45, 50, 60, 70, 80, 90, 100, 110, 120, 130, 140, 150, 160 or 170 amino acid residues of the G-CSF protein. Preferably said fragments are biologically active fragments, i. e. fragments which have G-CSF activity. For example, said fragments are capable to, e.g., mobilize multipotent stem cells, improve cardiac function or reduce mortality after acute myocardial. Particularly preferred G-CSF activity can be determined by its ability to induce colony formation, as reported in the literature by Bodine (1993), Blood 82:445-55; Bodine (1994), Blood 84, 1482-91.

In accordance with the present invention the term "pharmaceutical composition" relates to a composition comprising G-CSF or fragment thereof.

Such pharmaceutical compositions comprise a therapeutically effective amount of G-CSF or fragment thereof and, optionally, a pharmaceutically acceptable carrier. The pharmaceutical composition may be administered with a physiologically acceptable carrier to a patient, as described herein. In a specific embodiment, the term "pharmaceutically acceptable" means approved by a regulatory agency or other generally recognized pharmacopoeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium ion, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the aforementioned compounds, preferably in purified form, together with a suitable amount of carrier so as to provide the form for proper administration to the patient. The formulation should suit the mode of administration.

Preferably, the pharmaceutical composition of the present invention is suitable for administration to a patient. In the context of the present invention the term "patient" means an individual in need of a treatment of organ defects and/or dysfunction caused by ischemia. Preferably, the patient is a vertebrate, even more preferred a mammal, particularly preferred a human.

In another preferred embodiment, the composition is formulated in accordance with routine procedures as a pharmaceutical composition adapted for intravenous administration to mammals, preferably vertebrates and more preferably human beings. Typically, compositions for intravenous administration are solutions in sterile isotonic aqueous buffer. Where necessary, the composition may also include a solubilizing agent and a local anesthetic such as lignocaine to ease pain at the site of the injection. Generally, the ingredients are supplied either separately or mixed together in unit dosage form, for example, as a dry lyophilised powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The pharmaceutical composition of the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

In vitro assays may optionally be employed to help identify optimal dosage ranges. Effective doses may be extrapolated from dose-response curves derived from in vitro or animal model test systems. The precise dose to be employed in the formulation will also depend on the route of administration, and the seriousness of the disease or disorder should be decided according to the judgment of the practitioner and each patient's circumstances. Moreover, for example, the following factors concerning the precise dose may also be taken into account: patient's size, body surface area, age, sex, general health, and other drugs being administered concurrently.

Therefore, it is well known in the art that the dosage regimen will be determined by the attending physician and clinical factors. Proteinaceous pharmaceutically active matter may be present in amounts between 1 ng and 10 mg/kg body weight per dose; however, doses below or above this exemplary range are envisioned, especially considering the aforementioned factors.

Regimes known in the art to be capable of mobilizing increased numbers of bone-marrow derived stem cells into the blood circulation are commonly known in the art.

For example, 5 to 15 µg/kg of body weight of G-CSF for a total of 3 to 5 days in generally effective in inducing elevated levels of stem cells

The dosage regimen of G-CSF which is to be administered to the patient ranges preferably from 0.1 to 100 µg per kg body weight, more preferably from 1 to 100 µg per kg body weight, even more preferably from 1 to 50 µg per kg body weight d. s. c. over a period of at least 1 day, preferably at least 2 days, more preferably at least 3 days, even more preferably at least 4 days and particularly preferred at least 5 days. However, any other suitable dosage regimen is envisaged which may be determined as described herein.

The administration of the candidate agents of the present invention can be done in a variety of ways, including, but not limited to, orally, subcutaneously, intravenously, intranasally, intrabronchially, transdermally, intranodally, intradermally, intrarectally, intraperitoneally, intramuscularly, intrapulmonary, intraocularly, vaginally, rectally or topically. Where necessary, the composition may also include a solubilizing agent and a local anesthetic such as lignocaine to ease pain at the site of the injection. Generally, the ingredients are supplied either separately or mixed together in unit dosage form, for example, as a dry lyophilised powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration

In a particularly preferred embodiment the pharmaceutical composition comprising G-CSF or fragment thereof is administered subcutaneously. In another preferred embodiment the pharmaceutical composition comprising G-CSF or fragment thereof is administered via routes of administration as described supra and infra.

The terms "treatment" and "treating" are used herein to generally mean obtaining a desired pharmaceutical and/or physiological effect. Preferably, the effect is therapeutic in terms of partially or completely curing ischemia. The term "treatment" as used herein covers any treatment of organ defects and/or dysfunction caused by ischemia in a mammal, particularly a vertebrate and more preferably a human, and includes regenerating and/or repairing organ or tissue dysfunction.

The term "ischemia" as used herein relates to a condition that may occur in any organ that is suffering a lack of oxygen supply and/or supply with metabolites which occurs when there is an imbalance between oxygen supply and demand, due to inadequate perfusion, e.g., caused by atherosclerosis, restenoic lesions, anemia, stroke or clogged arteries just to name a few , that leads to insufficient oxygen to tissues such as, for example, the heart or brain. However, also medical interventions such as the interruption of the blood flow, e.g., during bypass surgery may lead to ischemia. Said term also encompasses the two most common types of ischemia; i.e. cardiac ischemia and cerebral ischemia. Cardiac ischemia includes a broad variety of conditions, from silent ischemia to stable or unstable angina to myocardial infarction (AMI or "heart attack"). Cerebral ischemia includes prolonged cerebral ischemic syndromes to completed stroke or cerebral infarction. However, ischemia is not limited to the aforementioned organs or tissues, respectively, since it may occur in any organ that is suffering a lack of oxygen supply and/or supply with metabolites.

The term "surgical or interventional procedure" as used herein relates to a surgical and/or interventional procedure which is suitable to improve organ function, to improve blood flow in defect organ tissue and/or to induce revascularization as thrombolysis (either systemic or local via catheter delivery), ballon angioplasty, stenting, coronary, carotoid or peripheral bypass surgery, endotherctomy or ventriculo-coronary stenting.

The present invention also relates to a method of treating organ dysfunction caused by ischemia comprising administering a therapeutically effective amount of G-CSF or fragment thereof to a patient who is subjected to a surgical or interventional procedure in order to improve organ function, to improve blood flow and/or to induce revasularization.

The term "administered" means administration of a therapeutically effective dose of G-CSF or fragment thereof to a patient. Preferably, said therapeutically effective dose of G-CSF or fragment is administered to a patient who is subjected to a surgical or interventional procedure. Particularly preferred said therapeutically effective dose is administered to a patient suffering from organ dysfunction caused by ischemia.

By "therapeutically effective amount" is meant a dose that produces the effects for which it is administered. The exact dose will depend on the purpose of the treatment, and will be ascertainable by one skilled in the art using known techniques. As is known in the art and described above, adjustments for systemic versus localized delivery, age, body weight, general health, sex, diet, time of administration, drug interaction and the severity of the condition may be necessary, and will be ascertainable with routine experimentation by those skilled in the art.

The methods are applicable to both human therapy and veterinary applications. The compounds described herein having the desired therapeutic activity may be administered in a physiologically acceptable carrier to a patient, as described herein. Depending upon the manner of introduction, the compounds may be formulated in a variety of ways as discussed below. The concentration of therapeutically active compound in the formulation may vary from about 0.1-100 wt %. The agents may be administered alone or in combination with other treatments.

The administration of G-CSF or fragment thereof can be done in a variety of ways as discussed above.

The attending physician and clinical factors will determine the dosage regimen asa described herein. A typical dose can be, for example, in the range of 0.001 to 1000 µg; and as described supra however, doses below or above this exemplary range are envisioned, especially considering the aforementioned factors.

The dosages are preferably given once or twice a day for the period of 5 days, however, during progression of the treatment the dosages can be given in much longer time intervals and in need can be given in much shorter time intervals, e.g., daily divided into multiple applications. If the regimen is a continuous infusion, it should also be in the range of 1 µg to 10 mg units per kilogram of body weight per minute, respectively. Progress can be monitored by periodic assessment. It is also envisaged that the pharmaceutical compositions are employed in co-therapy approaches, i.e. in co-administration with other medicaments or drugs, for example other drugs for preventing, treating or ameliorating ischemia. It is also preferred that the pharmaceutical composition of the present invention is co-administered with GM-CSF (granuloyte macrophage colony stimulating factor), SCF (stem cell factor), IL-3 (interleukin-3) and/or IL-6 (interleukin-6) or fragment thereof. This means that G-CSF or fragment thereof may be administered in combination with one or more of the aforementioned substances.

The findings of the present invention that G-CSF can be used for the effective treatment of ischemia in a patient who is subjected to surgical measure or intervention are even more intriguing since an NIH clinical research study (02-H-0264) in which G-CSF is administered to patients with coronary artery disease excludes patients who have had myocardial infarction within the last two months or have acute coronary syndromes, such as crescendo anginas. Moreover, the positive effects of G-CSF could not have been expected since G-CSF also has the effect of increasing the number of leucocytes which, concomitantly, leads to leukocyte and/or thrombocyte activation potentially resulting in deterioration of myocardial function, i.e. caused by ischemia-reperfusion injury. In dog experiments granulocytes (or polymorphonuclear leukocytes, PMN) caused reperfusion ventricular dysfunction in ischemic myocardium (Engeler (1987) Circ. Res. 61: 20-28) and resulted in capillary plugging in myocardial ischemia reperfusion injury (Engeler (1983) Am. J. Pathol 111:98-111). Increased expression of neutrophil and monocyte adhesion molecules during myocardial infraction may contribute to their adhesion to endothelium in the ischemic territory. This adhesion could feasibly precipitate vasoconstriction or add a local thrombotic effect due to tissue factor expression secondary to Mac-1 engagement. In addition, the manifestation of increased density of LFA-1 and Mac-1 by activated leukocytes with monocytes also expressing ICAM-1 suggests that leukocytes may form microaggregates that could cause microvascular plugging. This mechanism may facilitate the occurrence of the "no-reflow" phenomenon or slow coronary filling after acute myocardial infarction (Meisel (1998), J. Am. Coll. Cardiol. 31: 120-125). It is known in the art that after initiation of G-CSF treatment, an in vivo PMN activation occurred, as demonstrated by the significant increment of surface CD11b/CD18 and plasma elastase antigen levels. Moreover, PMN cellular elastase activity, which was significantly increased at 1 day of treatment, returned to baseline at day 5 to 6, in correspondence with the elastase antigen peak in the circulation. This change is accompanied by a parallel significant endothelial and coagulation activation (Falanga (1999) Blood 93: 2506-2514). However, additional endothelial activation and thrombus formation should be avoided in patients suffering from myocardial infarction. However, blood clotting should be avoided in patients suffering from myocardial infarction. Thus, the benefits arising from the present invention could not have been expected

In a preferred embodiment of the invention the pharmaceutical composition of the present invention or the effective amount G-CSF or fragment thereof is to be administered before said surgical or interventional procedure.

The term "before said surgical or interventional measure" when used in the context of the present invention means that the pharmaceutical composition comprising G-CSF or fragment thereof is to be administered before one day, preferably two days, more preferably three days and even more preferably four days of said surgical or interventional measure, for example, to prevent ischemic disease as described herein. Particularly preferred, it is to be administered before 5 days.

In another further preferred embodiment of the invention the pharmaceutical composition of the present invention or the effective amount of G-CSF or fragment thereof is to be administered during said surgical or interventional procedure.

The term "during said surgical or interventional measure" when used in the context of the present invention means that the pharmaceutical composition comprising G-CSF or fragment thereof is to be administered during a surgical or interventional procedure.

In an also preferred embodiment of the invention the pharmaceutical composition of the present invention or the effective amount of G-CSF or fragment thereof is to be administered after said surgical or interventional procedure.

The term "after said surgical or interventional measure" when used in the context of the present invention means that the pharmaceutical composition comprising G-CSF or fragment thereof is to be administered preferably immediately after said surgical or interventional measure. Particularly preferred, it is to be administered after at least 1 hour, preferably after at least 6 hours and even more preferably after at least 12 hours.

In a particular preferred embodiment of the present invention the pharmaceutical composition of the present invention or the effective amount of G-CSF or fragment thereof is to be administered preferably between 1 hour and 10 days, more preferably between 1 hour and 7 days, even more preferably between 1 hour and 5 days and particularly preferred between 2 hours and 5 days after said surgical or interventional procedure. As it is demonstrated in the appended Examples administration of a G-CSF after, for example, infarction has great beneficial effects to patients.
Preferably, the ischemia treated with the pharmaceutical composition or G-CSF fragment thereof of the present invention is selected from the group consisting of myocardial ischemia, cerebral ischemia, renal ischemia, liver ischemia, peripheral muscle tissue ischemia, retinal ischemia and spinal cord ischemia. As described supra, ischemia may occur in any organ suffering from a lack of oxygen and/or metabolites for a prolonged time which results in organic defects. The term "organ defect" as used herein relates to dysfunctional myocardium, brain, kidney, liver, peripheral muscle, retina or spinal cord defects. Said organ defects are causedby myocardial ischemia, e.g., due to hypertension, coronary artery disease (CAD), myocardial infarction, thrombo-embolic events, trauma and/or surgical procedures; cerebral ischemia, e.g., due to trauma, stroke, thrombo-embolic events, malformation of blood-supplying vessels, multi-infarct disease, cerebral hemorhage, surgical and/or interventional measures; renal ischemia, e.g., due to thrombo-embolic events, atherosclerosis, malformation of blood-supplying vessels, trauma and/or surgical procedures; liver ischemia, e.g., due thrombo-embolic events, malformation of blood-supplying vessels, trauma and/or surgical procedures; peripheral muscle tissue ischemia, e.g., is caused by thrombo-embolic events, atherosclerosis, malformation of blood-supplying vessels, trauma and/or surgical procedures; retinal ischemia, e.g., is caused by thrombo-embolic events, malformation of blood-supplying vessels, trauma and/or surgical procedures; and spinal cord ischemia, e.g., is caused by thrombo-embolic events, atherosclerosis, malformation of blood-supplying vessels, trauma and/or surgical procedures.

It is particularly preferred that the myocardial ischemia which is treated with the pharmaceutical composition or effective amount of G-CSF or fragment thereof of the present invention is caused by hypertension, coronary artery disease (CAD), myocardial infarction, thrombo-embolic events, trauma and/or surgical procedures.

It is also particularly preferred that the cerebral ischemia which is treated with the pharmaceutical composition or effective amount of G-CSF or fragment thereof of the present invention is caused by trauma, stroke, thrombo-embolic events, malformation of blood-supplying vessels, multi-infarct disease, cerebral hemorhage, surgical and/or interventional measures.

In still another embodiment it is particularly preferred that the renal ischemia which is treated with the pharmaceutical composition or effective amount of G-CSF or fragment thereof of the present invention is caused by thrombo-embolic events, atherosclerosis, malformation of blood-supplying vessels, trauma and/or surgical procedures.

In a furthermore particularly preferred embodiment it is envisaged that the liver ischemia or retinal ischemia which is treated with the pharmaceutical composition or effective amount of G-CSF or fragment thereof of the present invention is caused by thrombo-embolic events, malformation of blood-supplying vessels, trauma and/or surgical procedures.

A still particularly preferred aspect of the present invention is that the peripheral muscle tissue ischemia or spinal cord ischemia which is treated with the pharmaceutical composition or method of treatment of the present invention is caused by thrombo-embolic events, atherosclerosis, malformation of blood-supplying vessels, trauma and/or surgical procedures.

In an even more particularly preferred embodiment the ischemia which is treated with the pharmaceutical composition or effective amount of G-CSF or fragment thereof of the present invention causes organ defects.

It is also preferred that the surgical or interventional procedure of the present invention to which a patient is subjected to is a procedure to improve organ function, to improve blood flow and/or to induce resvascularization selected from the group consisting of thrombolysis, ballon angioplasty, stenting, coronary or peripheral bypass surgery and ventriculo-coronary stenting. These techniques are commonly known in the art to the person skilled in the art.

Moreover, it is preferred that the pharmaceutical composition of the present invention comprising G-CSF or fragment thereof is capable of recruiting stem and/or progenitor cells.

The term "recruiting stem and/or progenitor cells" as used herein refers to the ability to attract stem and/or progenitor cells from, e.g. the bone marrow towards the damaged organ and/or tissue. Without being bound by theory, it is assumed that also SDF-1 is required to induce stem cell homing to injured tissue, e.g., myocardium. Stem cells may bind SDF-1 via their CXCR4 receptor (Petit (2002) Nat. Immunol. 687-694). Preferably, said stem and/or progenitor cells are selected from the group consisting of CD34(+), multipotent adult progenitor cells (MAPC), endothelial progenitor cells (EPC), side population cells (SP) and lineage-negative stem cells. The stem cells and/or progenitor cells are characterized by using FACS analysis as described in the appended Examples.
More preferably, said multipotent adult progenitor cells are CD34(-), vascular endothelial cadherin(-) and AC133(+) and Flk1(+) and said endothelial progenitor cells are preferably CD34(+), CD31 (+) and KDR(+).

Said cells of the side population are preferably CD34(-) / low, c-Kit(+) or Sca-1 (+) and said lineage-negative stem cells are CD5(-), CD19(-),CD34(-), c-Kit(+) and Sca-1(+). The characterization of stem and/or progenitor cells is shown in the appended Examples infra.

The indication "(+)" and "(-)" refer to results obtained by flow cytometry (FACS) analysis. (+) or "positive" means that a defined protein, such as CD34, CD31, etc. is expressed on the surface of an analyzed cell, such as a stem cell or progenitor cell, etc. (-) or "negative" means that a defined protein, such as CD34, CD31 etc. is not expressed on the surface of an analyzed cell, such as a stem cell or a progenitor cell, etc.

Thus, it was surprisingly found that besides hematopoetic stem cells, G-CSF results in mobilization of other populations of stem cells with a potential of myocardial and/or endothelial repair and regeneration.

In a more preferred embodiment said stem and/or progenitor cells home to organs which harbour defects due to ischemia. Preferably, said stem and/or progenitor cells are capable of repairing or regenerating said organs.

The term "homing" as used herein refers to the stem and/or progenitor cells' innate ability to travel preferably to the right place in the body. Preferably, said stem and/ or progenitor cells travel to sites where organ defects/ dysfunction caused by ischemia have taken/take place. The terms "repairing" and "regenerating" as used herein relates to restore at least partially the former conditions of the defect organ tissue.

The Figures show:

**Figure 1**: This figure illustrates the design of G-CSF in STEMI trial. G-CSF: granulocyte colony-stimulating factor, STEMI: ST segment elevation myocardial infarction. PTCA: percutaneous transluminal coronary angioplasty, s.c.: subcutaneously, MRI: magnetic resonance imaging, TTE: transthoracic echocardiography, AE: Adverse event., Lab.: Laboratory investigation.

**Figure 2**: This figure describes the Inclusion criteria of G-CSF in STEMI trial. MI: myocardial infarction, ECG: electrocardiography.

**Figure 3**: This figure shows adverse events occurred during study period (18 patients enrolled). AE: adverse event. *: patient was unbonded and identified to receive G-CSF; §: patient was unblinded and identified to receive placebo.

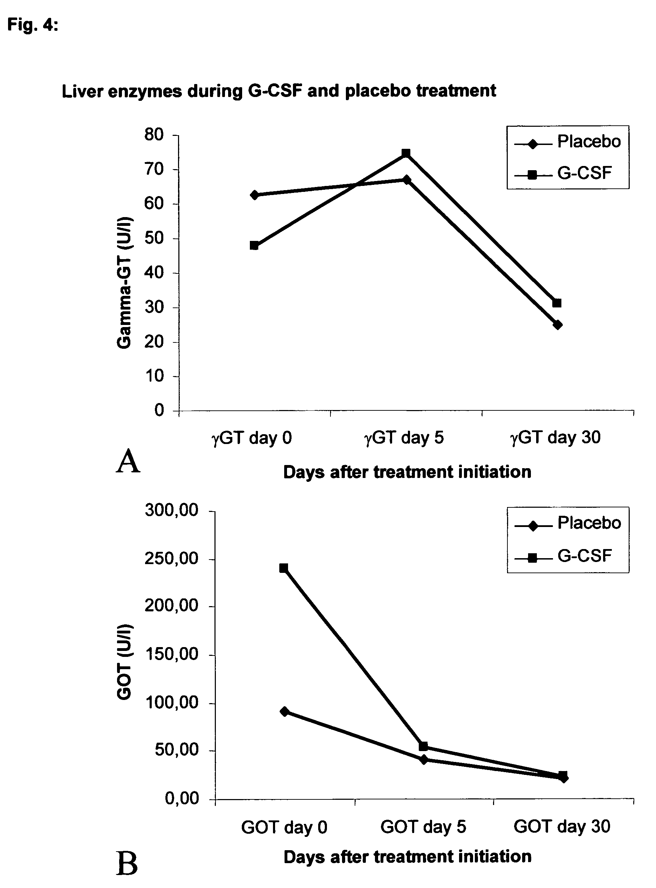

**Figure 4**: This figure shows liver enzymes during G-CSF or placebo treatment. GammaGT: Gamma-Glutaryl-Transferase. GOT:

Glutamat-oxalat transaminase. U/L: unit per liter.

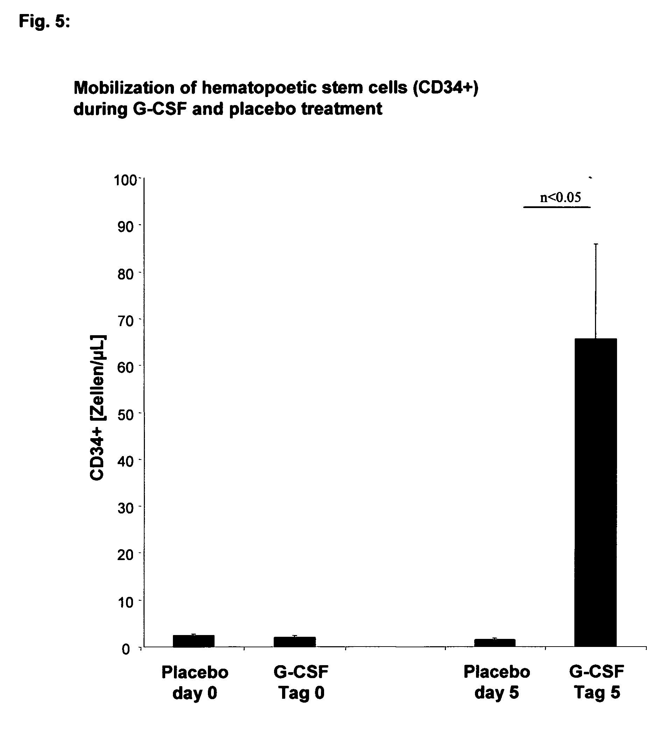

**Figure 5**: This figure illustrates mobilization of hematopoetic stem cells (CD34+) during G-CSF and placebo treatment.

**Figure 6**: This figure illustrates mobilization of other stem cell populations during G-CSF and placebo treatment.

A: Comparison of endothelial progenitor cells (EPC, CD34+/CD31+) in different treatment groups at day 0 and day 5 after therapy (CD34+/CD31+) of total white blood count (WBC).
B: EPC population in placebo patient at day 5,
C: Comparison of percentage of different stem cell populations (in relation to total white blood count) after stem cell mobilization using G-CSF at day 5.
D-G: Different stem cell populations such as EPC (D), c-kit+ with or without expression of CD34+ (E), MAPC (F), and side population (G) before (left) and at day 5 of treatment with G-CSF (right).

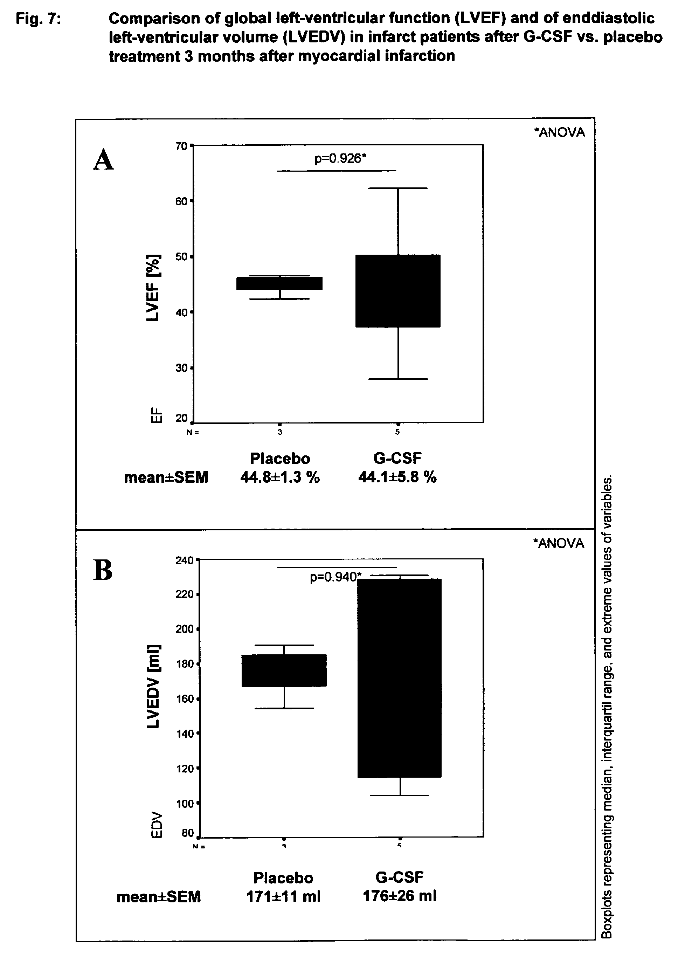

**Figure 7**: This figure illustrates the comparison of global left-ventricular function parameters after G-CSF vs. placebo treatment 3 months after myocardial infarction assessed by magnetic resonance imaging. A: Left-ventricular ejection fraction (LVEF), B: Enddiastolic left-ventricular volume (LVEDV). Statistical analysis by analysis of variance (ANOVA). SEM: standard error of the mean.

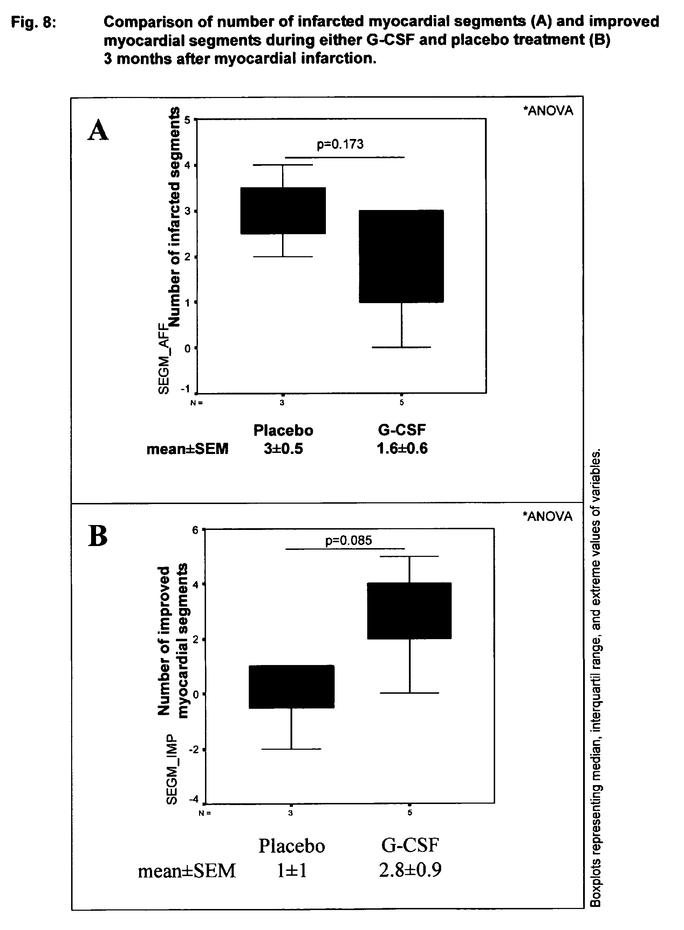

**Figure 8**: This figure shows the comparison extent of myocardial damage at 3 months assessed by magnetic resonance imaging. A: number of infarcted myocardial segments B: improved myocardial segments during either G-CSF and placebo treatment 3 months after myocardial infarction. Statistical analysis by analysis of variance (ANOVA). SEM: standard error of the mean.

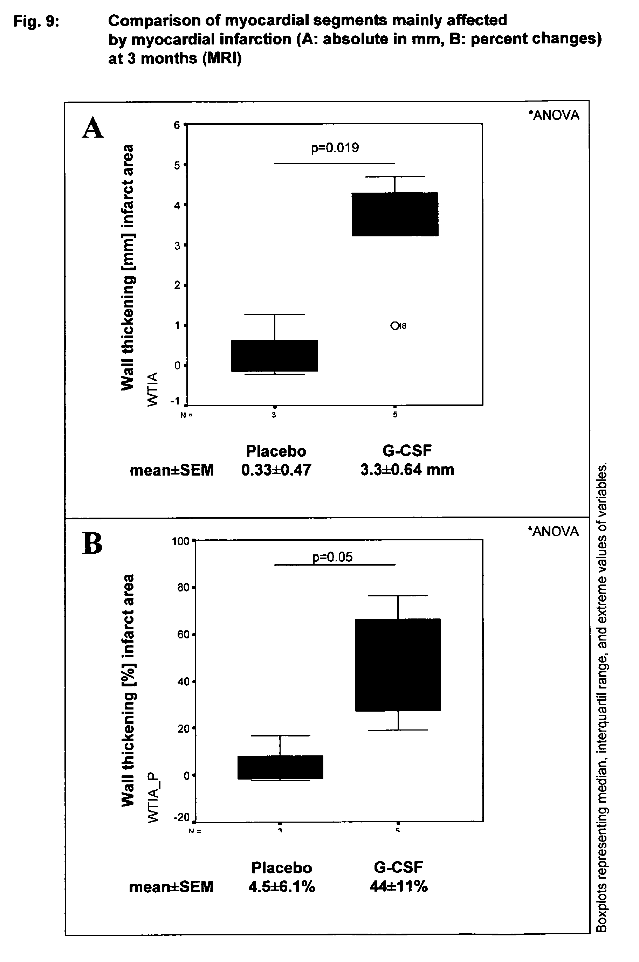

**Figure 9**: This figure illustrates the comparison of regional wall motion in segments mainly affected by myocardial infarction assessed by magnetic resonance imaging. A: Ventricular wall thickening of the infarct area in mm. B: Ventricular wall thickening of the infarct area in percent during either G-CSF and placebo treatment 3 months after myocardial infarction. Statistical analysis by analysis of variance (ANOVA). SEM: standard error of the mean.

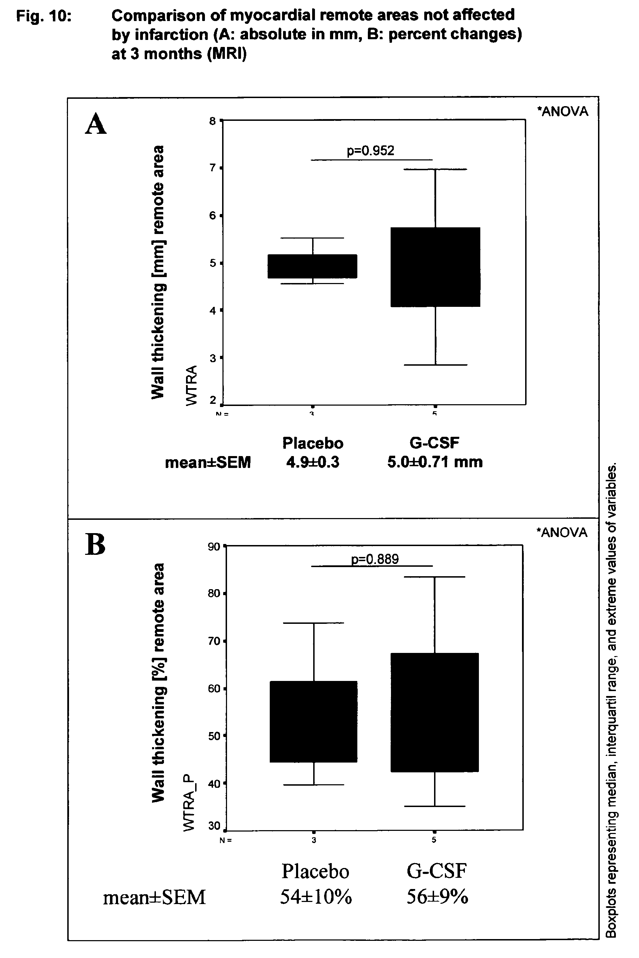

**Figure 10:** This figure shows the comparison of regional wall motion in segments not affected by myocardial infarction assessed by magnetic resonance imaging.
A: Ventricular wall thickening of the romote area in mm. B: Ventricular wall thickening of the remote area in percent during either G-CSF and placebo treatment 3 months after myocardial infarction. Statistical analysis by analysis of variance (ANOVA). SEM: standard error of the mean.

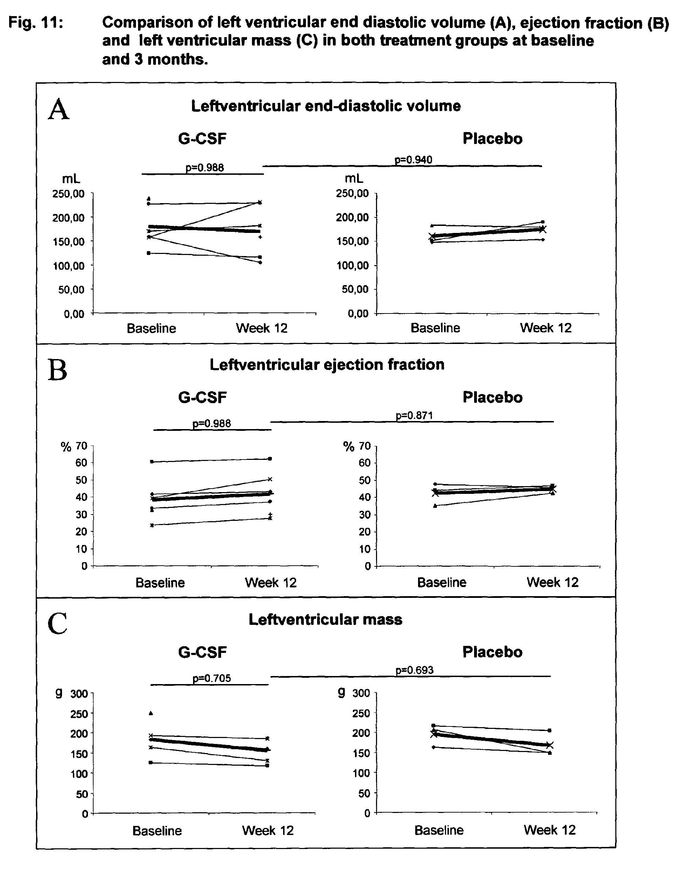

**Figure 11:** This figure illustrates the comparison of global ventricular function parameters between both treatment groups assessed by magnetic resonance imaging at baseline and 3 months after myocardial infarction.
A: Enddiastolic left-ventricular volume (LVEDV). B: Left-ventricular ejection fraction (LVEF), t 3 months after myocardial infarction. C: Left-ventricular mass. Statistical analysis by analysis of variance (ANOVA). SEM: standard error of the mean.

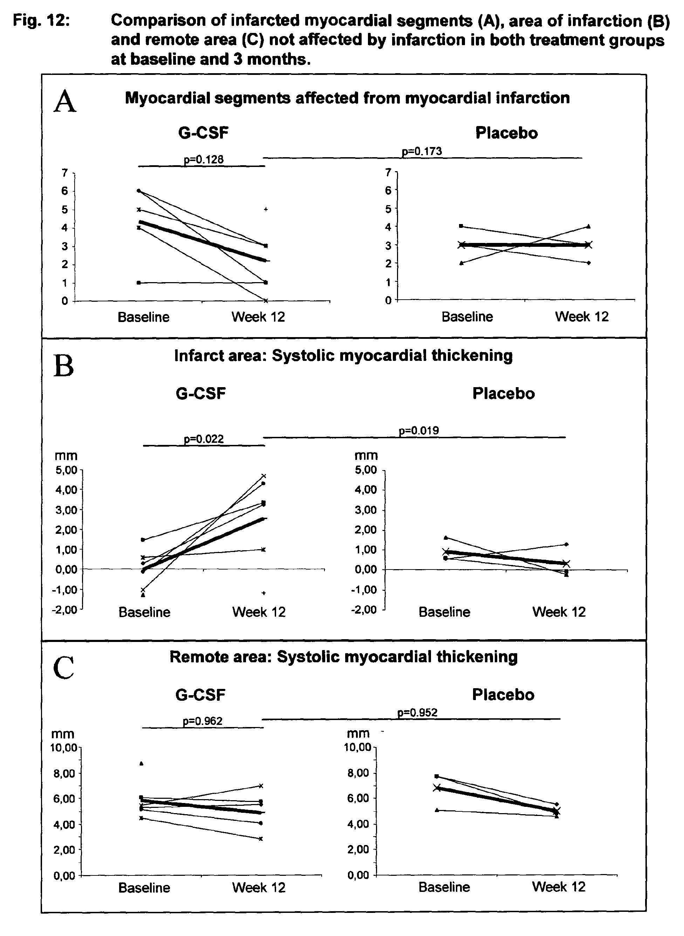

**Figure 12:** This figure shows the comparison of reginal ventricular function parameters between both treatment groups assessed by magnetic resonance imaging at baseline and 3 months after myocardial infarction.
A: Myocardial segments mainly affected from myocardial infarction. B: Ventricular wall thickening of the infarct area in mm., t 3 months after myocardial infarction. C: Left Ventricular wall thickening of the remote area in mm. Statistical analysis by analysis of variance (ANOVA). SEM: standard error of the mean.

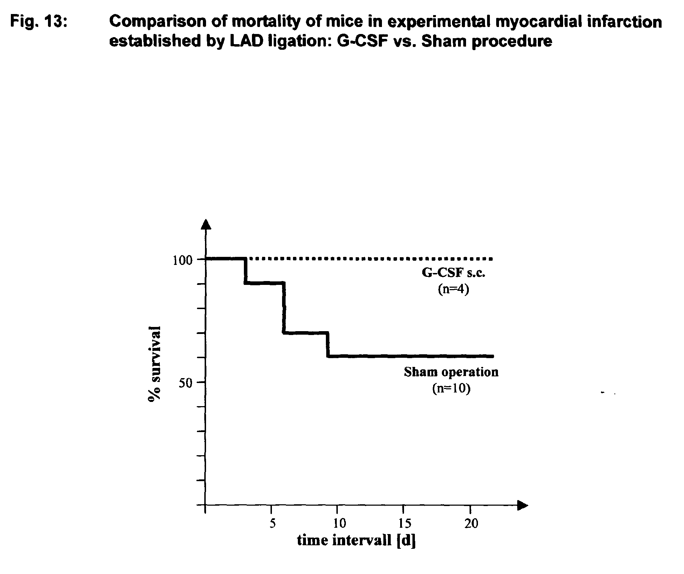

**Figure 13:** This figure shows the comparison of survival in mice with experimental myocardial infarction receiving either G-CSF after infarction or sham procedure.

The Examples illustrate the invention:

**Example 1**: Design of the clinical study

The primary objective of this study is to assess myocardial regeneration by peripheral blood stem cells mobilized by G-CSF, as measured by change of myocardial thickness, regional myocardial function and global cardiac function from baseline over 12 weeks of follow-up using cardiac magnetic resonance tomography (MRI). In addition, the extent of non-viable myocardium will be monitored from baseline up to 12 weeks. Analysis of cardiac function consists of evaluation of end-diastolic myocardial thickness, segmental systolic wall thickening, end-diastolic volume (EDV), end-systolic volume, stroke volume, ejection fraction (EF) and cardiac output.

Secondary endpoints comprised changes from baseline in the following parameters over 12 weeks of follow-up between treatment groups: occurrence of major adverse cardiac events (death, myocardial infarction, CABG, or re-intervention), reported spontaneously reported adverse events (AEs), hematological changes (leukocyte, erythrocyte and platelet counts), number and characterization of mobilized stem cells.

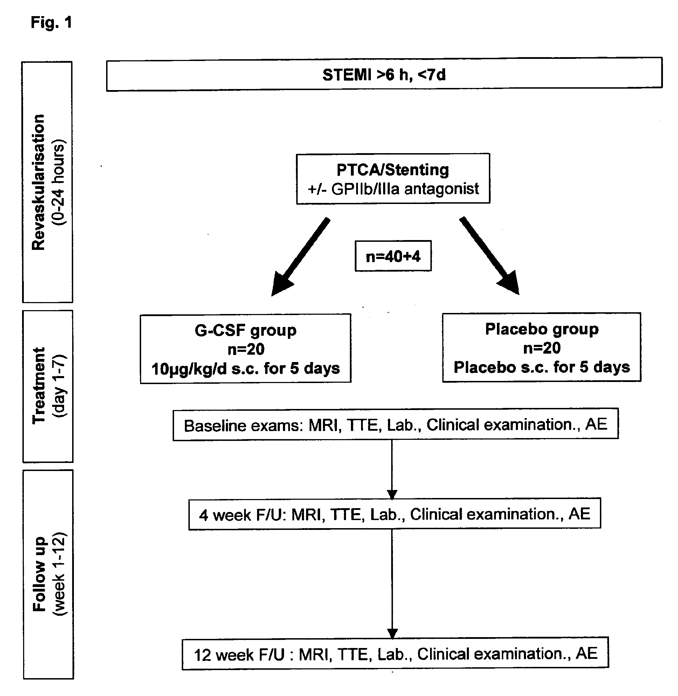

Accordingly, this Phase I, single center, randomized, controlled efficacy and safety study compares the effects of a 5 day treatment of G-CSF administered s.c. in patients with acute ST segment elevation myocardial infarction of more than 6 hours and less than 7 days duration since initial symptoms who are candidates for percutaneous coronary revascularization procedure. The study consisted of a *Revascularization Period* (angioplasty of the infarcted vessel), a *Treatment Period* (up to 6 days), and a Follow-up Period (12 weeks). The *Revascularization Period* started with the treatment of the patient in the emergency room, administering aspirin, heparin, betablockers, and nitrates as appropriate and not done by the transferring emergency physician. As soon as possible the patient will be transferred to the catheterization laboratory where acute angioplasty and stenting of the infarcted artery was performed. After stent placement, the patient was treated with clopidogrel. If appropriate, the patient will be treated with glycoprotein IIb/IIIa antagonists. After evaluating patient's eligibility to enter the study patients were randomized for the control or G-CSF treatment group. First 4 patients received G-CSF (Filgrastim, Amgen GmbH, Munich, Germany) at a dose of 10µg/kg body weight/day s.c. divided into 2 applications to investigate safety and adverse reactions in patients suffering from myocardial infarction. The following patients were randomized to receive either G-CSF or placebo. Follow-up assessment will be performed in all patients at week 4 and week 12. The overall schedule of time and events is shown in Figure 1. Inclusion and exclusion criteria, follow-up examinations are documented in the attached study protocol. The study was approved by the university's ethics committee and all patients undersigned a written informed consent.

**Example 2**: Safety of administration of G-CSF

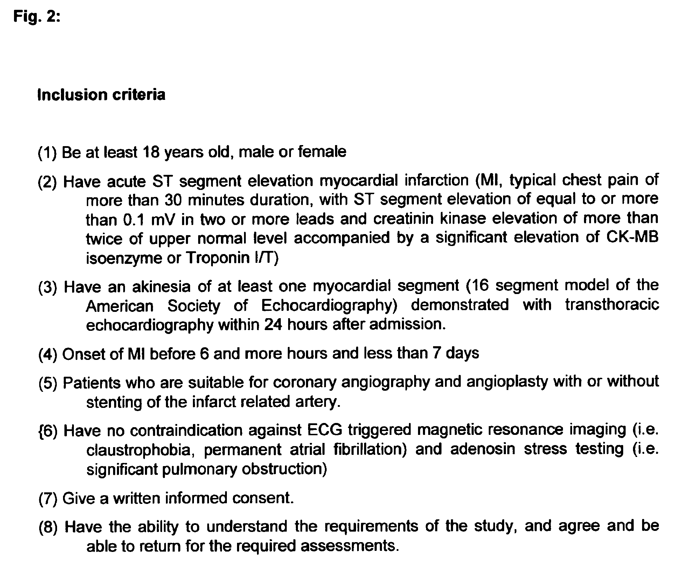

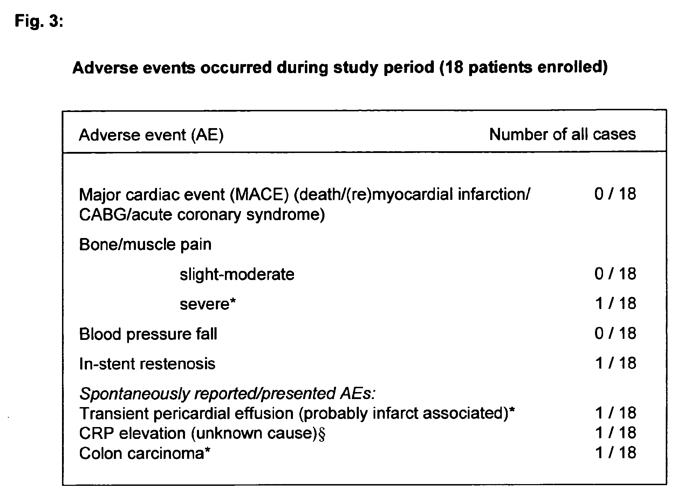

Study design and inclusion criteria are shown in Fig. 1 and 2. A pilot phase from December 2002 to February 2003 was achieved to test safety of G-CSF induced stem cell mobilization in patients suffering from acute myocardial infarction. Within this pilot phase 4 male patients (mean age 62±13 years received G-CSF (Neupogen®) 10 µg/kg/d s.c. over 5 days. All patients suffered from extensive myocardial infarction (CK max around 2000 IU/L) that was treated using primary PCI a mean of 12 hours after onset of infarction. Coronary intervention (stent) was successful achieved in all patients. White blood count increased from 11±3 G/L to 50±25 G/L and number of mobilized hematopoetic stem cells (CD34+) increased to 54±24/µL (20fold increase). Renal and liver function tests were unchanged between both therapy groups. No serious adverse events such as myocardial (re)infarction, coronary syndrome or death or coronary artery bypass grafting were observed (see Fig. 3). After showing safety of the treatment using G-CSF, the randomization period started in March 2003. Since March 2003, another 14 patients were enrolled in the study (12 male, 2 female). Mean time from onset of infarction to primary coronary intervention (PCI) was 30 hours. Myocardial damage was severe (mean CK max. 3500 IU/L). In all patients a coronary intervention with stent placement was possible and successful. Adverse events occurred were the following: moderate to severe bone and muscle pain in one patient during application of the study drug (unblinded: G-CSF), the AE resolved after termination of the study drug. The AE was coincident with a concommittant common-cold of the patient. One patient suffered from in stent restenosis and could be treated using ballon angioplasty. One female patient developed a hemodynamically non-relevant pericardial effusion that disappeared completely after termination of the study drug. One patient of control group had elevated C-reactive protein of unknown origin which resolved completely after administration of steroids. Another patient of the G-CSF group presented anemia and stool abnormalities during outpatient follow-up examination. The diagnosis was a formerly undiagnosed colon carcinoma, patient was treated with combined chemo- and radiation therapy.

**Example 3**: Magnetic resonance imaging

The MRI examinations were carried out on a 1.5 T whole body scanner (Magnetom Symphony, Siemens Medical Systems, Erlangen, Germany) equipped with state-of-the art cardiac software and dedicated array coil systems for signal optimization. The functional examination is based on a shared segmented CINE TrueFISP technique with a temporal resolution of <40ms and a spatial resolution of 1-2mm at a slice thickness of 5-7mm. Functional examinations were performed according to the following scheme: Based on adjustment to the patients individual cardiac axis data sets were acquired along the cardiac long axis (vertical long axis), the four chamber view (horizontal long axis) and a stack of views along the short axis. To identify myocardial segments the myocardial circumference were divided into 6 segments within the short axis view using the anterior junction of the right ventricle as a reference. For assessing regional wall motion a 16-segment model was used. The cardiac volumes and the regional functional parameters were assessed using a dedicated cardiac post-processing software. This software allows a semi-automated segmentation of the endo- and epicardial border. To calculate EDV and ESV the stack of short axis images will be segmented at end-diastole and end-systole and the volume computation performed using the Simpsons' rule. The derived parameters SV, EF and cardiac output (CO) were calculated as follows: SV=EDV-ESV; EF=(EDV-ESV)/EDV; CO=SV x heart rate. To assess regional systolic myocardial thickening the difference between the end-diastolic and end-systolic thickness were calculated on a segmental base as absolute and relative values.

The myocardial viability was assessed using T1 w contrast enhanced MRI using the "late enhancement" technique. Strong T1w techniques will be applied along the intrinsic cardiac axes as described previously (cardiac function). For data sampling an inversion recovery TurboFLASH or TrueFISP technique were be used with a spatial resolution of 1-2mm and a slice thickness of 5-7 mm. To differentiate viable and non-viable myocardium the data acquisition follows the injection of gadolinium chelates (Multihance, Bracco Byk Gulden, Konstanz, Germany). A minimum delay of 10 min was necessary between the last contrast injection and the data acquisition. The volume of non-viable myocardium will be assessed by tracing enhanced myocardial areas in all consecutive short axis images, summed up and multiplied with the singe slice thickness.

MR data were analyzed by two independent experienced radiologists who were unaware of the therapeutic regime.

In summary, using MRI, 8 patients (G-CSF n=5, placebo n=3) who completed the follow up could be analyzed. Global myocardial function was unchanged in both treatment groups after 3 months (LV-ejection fraction, Placebo: 44.8±1.3% vs. G-CSF: 44.1±5.8%, p=0.926), ventricular volumes were moderately enlarged but did not differ in both groups (LVEDV, Placebo: 171±11 ml vs. G-CSF: 176±26 ml, p=0.940 Fig. 7). The number of infarcted myocardial segments improved at 3 months was increased (Placebo 1±1 vs. G-CSF 2.8±0.9, p=0.085, Fig. 8). The regional wall thickening of the main infarct area was significantly improved in patients receiving G-CSF treatment (Placebo: 0.33±0.47 mm vs. G-CSF: 3.3±0.64 mm, p=0.019, Fig. 9). Improvement of regional wall motion was nearly 60% of normal myocardium during systole in comparison to placebo treated patients, Fig. 9 and 10).

Thus, in conclusion, stem cell mobilization using G-CSF in patients suffering from myocardial infarction is feasible and safe. Adverse events are rare.

Moreover, the regional myocardial function is significantly improved in G-CSF treated patients in the long term.

**Example 4**: Mobilization of different stem cell populations analysed by flow cytometry

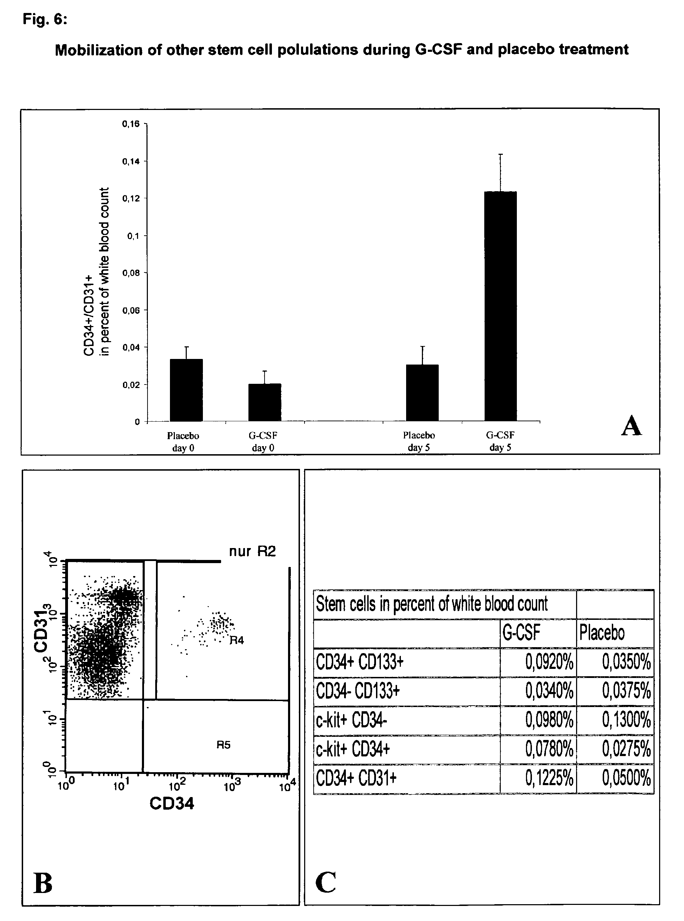

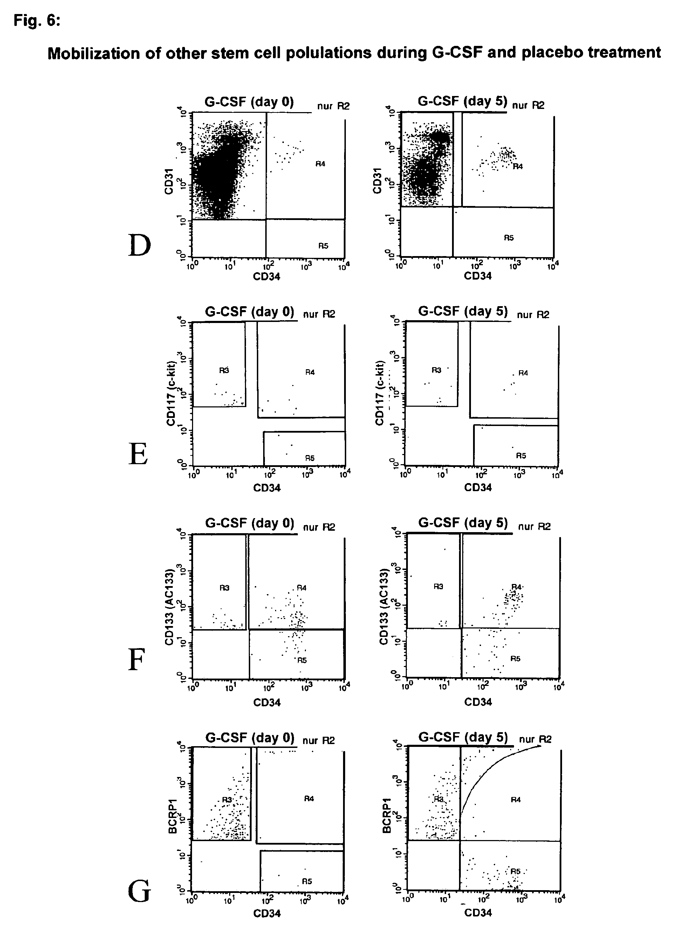

Immunophenotyping was performed with the following monoclonal antibodies conjugated with fluorescein isothiocyanate (FITC), phycoerythrin (PE), or phycoerythrin cyanine 5 (PE-Cy5): CD14, CD34, CD45, CD117, CD133, (BD PharMingen/ Coulter Immunotech, Hamburg, Germany). A volume of 100 µL of peripheral blood was incubated for 10 minutes in the dark with specific directly conjugated antibodies antibodies. After adding PeliLyse (Hiss Diagnostics GmbH, Freiburg, Germany) for 10 minutes, cells were washed in PBS and 0.1% sodium azide, centrifuged and resuspended in 200 µL PBS for FACS-analysis. Flow cytometric analysis was performed with a BD FACScan flow cytometer (Becton Dickinson, Heidelberg, Germany). Each analysis included 100 000 events. Stem cell populations were defined as the following: (1) multipotent adult progenitor cells (MAPC): CD34(-), AC133(+), Flk1(+) (Reyes (2002) *J Clin Invest*. 109:337-346). (2) endothelial progenitor cells (EPC): CD34 (+), CD31 (+) (Asahara (1997) *Science.* 275:964-967). (3) cells of the so-called *side population* (SP): CD34 (-)/low, c-Kit (+), Sca-1 (+) which also express BCRP-1. (4) so-called lineage-negative stem cells have the following markers CD95 (-), CD19 (-),CD34 (-),c-Kit (+), Sca-1 (+).
Using G-CSF, a variety of different stem cell populations other than CD34+ were mobilized. These progenitor cell populations consisted of EPC, MAPC, SP cells and lineage-negative c-kit+ cells (see Figure 6 A-G).
Surprisingly we found that stem cell populations having potential of repairing myocardial tissue are mobilized using G-CSF treatment.

**Example 5**: Animal model of myocardial infarction

Immunocompetent mice (n=14) were operated using standard techniques (anaesthesia, ventilation, thoracotomy). After ligation of the left anterior descending artery, animals received either G-CSF 50µg/kg via intraperitoneal injection over 5 days (n=4) or sham procedure (n=10) (see Figure 13).
It was surprisingly found that in mice, G-CSF when administered after experimental myocardial infarction, survival of animals was improved.

**Example 6**: Statistical analysis

Results are expressed in means±SE. One factorial ANOVA was chosen for analysis of normal distributed parameters. Non-parametrical tests included Kruskal-Wallis test, or Fisher's exact test where appropriate. A level of $p < 0.05$ was taken to indicate statistical significance (SPSS release 11.0.1, SPSS Inc., Chicago, IL).

**Claims**

1. Use of G-CSF or fragment thereof for the preparation of a pharmaceutical composition for the treatment of organ dysfunction caused by ischemia, whereby the pharmaceutical composition is to be administered to a patient who is subjected to a surgical or interventional procedure in order to improve organ function, to improve blood flow and/or to induce revascularization.

2. A method of treating organ dysfunction caused by ischemia comprising administering an effective amount of G-CSF or fragment thereof to a patient who is subjected to a surgical or interventional procedure in order to improve organ function, to improve blood flow and/or to induce revascularization.

3. The use of claim 1 or the method of claim 2, wherein said pharmaceutical composition or said effective amount of G-CSF or fragment thereof is to be administered before said surgical or interventional procedure.

4. The use of claim 1 or the method of claim 2, wherein said pharmaceutical composition or said effective amount of G-CSF or fragment thereof is to be administered during said surgical or interventional procedure.

5. The use of claim 1 or the method of claim 2, wherein said pharmaceutical composition or said effective amount of G-CSF or fragment thereof is to be administered after said surgical or interventional procedure.

6. The use or the method of claim 5, wherein said pharmaceutical composition or said effective amount of G-CSF or fragment thereof is to be administered between 2 hours and 5 days after said surgical or interventional procedure.

7. The use or the method of any one of claims 1 to 6, wherein said ischemia is selected from the group consisting of myocardial ischemia, cerebral ischemia, renal ischemia, liver ischemia, peripheral muscle tissue ischemia, retinal ischemia and spinal cord ischemia.

8. The use or the method of claim 7, wherein said myocardial ischemia is caused by hypertension, coronary artery disease (CAD), myocardial infarction, thrombo-embolic events, trauma and/or surgical procedures.

**9.** The use or the method of claim 7, wherein said cerebral ischemia is caused by trauma, stroke, thrombo-embolic events, malformation of blood-supplying vessels, multi-infarct disease, cerebral hemorhage, surgical and/or interventional measures.

**10.** The use or the method of claim 7, wherein said renal ischemia is caused by thrombo-embolic events, atherosclerosis, malformation of blood-supplying vessels, trauma and/or surgical procedures

**11.** The use or the method of claim 7, wherein said liver ischemia is caused by thrombo-embolic events, malformation of blood-supplying vessels, trauma and/or surgical procedures.

**12.** The use or the method of claim 7, wherein said peripheral muscle tissue ischemia is caused by thrombo-embolic events, atherosclerosis, malformation of blood-supplying vessels, trauma and/or surgical procedures.

**13.** The use or the method of claim 7, wherein said retinal ischemia is caused by thrombo-embolic events, malformation of blood-supplying vessels, trauma and/or surgical procedures.

**14.** The use or the method of claim 7, wherein said spinal cord ischemia is caused by thrombo-embolic events, atherosclerosis, malformation of blood-supplying vessels, trauma and/or surgical procedures.

**15.** The use or the method of any one claims 1 or 7 to 14, wherein said ischemia causes organ defects.

**16.** The use or the method of any one of claims 1 to 6, wherein said surgical or interventional procedure is a procedure to regain blood flow selected from the group consisting of thrombolysis, ballon angioplasty, stenting, coronary or peripheral bypass surgery and ventriculo-coronary stenting.

**17.** The use or the method of any one of claims 1 to 16, wherein said pharmaceutical composition or said effective amount of G-CSF or fragment thereof is capable of recruiting stem and/or progenitor cells.

**18.** The use or the method of claim 17, wherein said stem cells are selected from the group consisting of CD34(+), multipotent adult progenitor cells (MAPC), endothelial progenitor cells (EPC), side population cells (SP) and lineage-negative stem cells.

**19.** The use or the method of claim 18, wherein said multipotent adult progenitor cells are CD34(-), vascular endothelial cadherin(-) and AC133(+) and Flk1(+).

**20.** The use or the method of claim 18, wherein said endothelial progenitor cells are CD34(+), CD31 (+) and KDR(+).

**21.** The use or the method of claim 18, wherein said cells of the side population are CD34(-)/ low, c-Kit (+) or Sca-1 (+).

**22.** The use or the method of claim 18, wherein said lineage-negative stem cells are CD5(-), CD19(-), CD34(-), c-Kit(+) and Sca-1 (+).

**23.** The use or the method of any one of claim 17 to 22, wherein said cells home to organs which harbour defects due to ischemia.

**24.** The use or the method of claim 23, wherein said cells are capable of repairing and/or regenerating said organs.

**Fig. 1**

**Fig. 2:**

**Inclusion criteria**

(1) Be at least 18 years old, male or female

(2) Have acute ST segment elevation myocardial infarction (MI, typical chest pain of more than 30 minutes duration, with ST segment elevation of equal to or more than 0.1 mV in two or more leads and creatinin kinase elevation of more than twice of upper normal level accompanied by a significant elevation of CK-MB isoenzyme or Troponin I/T)

(3) Have an akinesia of at least one myocardial segment (16 segment model of the American Society of Echocardiography) demonstrated with transthoracic echocardiography within 24 hours after admission.

(4) Onset of MI before 6 and more hours and less than 7 days

(5) Patients who are suitable for coronary angiography and angioplasty with or without stenting of the infarct related artery.

{6) Have no contraindication against ECG triggered magnetic resonance imaging (i.e. claustrophobia, permanent atrial fibrillation) and adenosin stress testing (i.e. significant pulmonary obstruction)

(7) Give a written informed consent.

(8) Have the ability to understand the requirements of the study, and agree and be able to return for the required assessments.

**Fig. 3:**

**Adverse events occurred during study period (18 patients enrolled)**

| Adverse event (AE) | Number of all cases |
|---|---|
| Major cardiac event (MACE) (death/(re)myocardial infarction/ CABG/acute coronary syndrome) | 0 / 18 |
| Bone/muscle pain | |
|     slight-moderate | 0 / 18 |
|     severe* | 1 / 18 |
| Blood pressure fall | 0 / 18 |
| In-stent restenosis | 1 / 18 |
| *Spontaneously reported/presented AEs:* | |
| Transient pericardial effusion (probably infarct associated)* | 1 / 18 |
| CRP elevation (unknown cause)§ | 1 / 18 |
| Colon carcinoma* | 1 / 18 |

**Fig. 4:**

Liver enzymes during G-CSF and placebo treatment

A

B

**Fig. 5:**

Mobilization of hematopoetic stem cells (CD34+)
during G-CSF and placebo treatment

**Fig. 6:**

**Mobilization of other stem cell polulations during G-CSF and placebo treatment**

A

B

| Stem cells in percent of white blood count | | |
|---|---|---|
| | G-CSF | Placebo |
| CD34+ CD133+ | 0,0920% | 0,0350% |
| CD34- CD133+ | 0,0340% | 0,0375% |
| c-kit+ CD34- | 0,0980% | 0,1300% |
| c-kit+ CD34+ | 0,0780% | 0,0275% |
| CD34+ CD31+ | 0,1225% | 0,0500% |

C

**Fig. 6:**

**Mobilization of other stem cell polulations during G-CSF and placebo treatment**

**Fig. 7:** **Comparison of global left-ventricular function (LVEF) and of enddiastolic left-ventricular volume (LVEDV) in infarct patients after G-CSF vs. placebo treatment 3 months after myocardial infarction**

**Fig. 8:** **Comparison of number of infarcted myocardial segments (A) and improved myocardial segments during either G-CSF and placebo treatment (B) 3 months after myocardial infarction.**

**Fig. 9:** Comparison of myocardial segments mainly affected by myocardial infarction (A: absolute in mm, B: percent changes) at 3 months (MRI)

**Fig. 10:** Comparison of myocardial remote areas not affected by infarction (A: absolute in mm, B: percent changes) at 3 months (MRI)

**Fig. 11:** Comparison of left ventricular end diastolic volume (A), ejection fraction (B) and left ventricular mass (C) in both treatment groups at baseline and 3 months.

**Fig. 12: Comparison of infarcted myocardial segments (A), area of infarction (B) and remote area (C) not affected by infarction in both treatment groups at baseline and 3 months.**

**Fig. 13:**   Comparison of mortality of mice in experimental myocardial infarction established by LAD ligation: G-CSF vs. Sham procedure

**European Patent Office**

**PARTIAL EUROPEAN SEARCH REPORT**

which under Rule 45 of the European Patent Convention shall be considered, for the purposes of subsequent proceedings, as the European search report

Application Number

EP 03 02 4526

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 1 327 449 A (CHUGAI PHARMACEUTICAL CO LTD) 16 July 2003 (2003-07-16) * the whole document * ----- | 1-24 | A61K38/19 A61P9/10 A61P25/00 A61P13/12 A61P1/16 |
| X | WO 01/94420 A (ITESCU SILVIU ; UNIV COLUMBIA (US)) 13 December 2001 (2001-12-13) * the whole document * ----- | 1-24 | |
| X | WO 02/099081 A (CHAJUT AYELET ; QUARK BIOTECH INC (US)) 12 December 2002 (2002-12-12) * the whole document * ----- | 1-24 | |
| X | US 2003/147862 A1 (BUSCHMANN IVO R  ET AL) 7 August 2003 (2003-08-07) * the whole document * ----- | 1-5,7-9, 15,16 | |
| X | DE 100 33 219 A (UNIV HEIDELBERG) 24 January 2002 (2002-01-24) * the whole document * ----- | 1-5,7,9, 14-16 | |

**TECHNICAL FIELDS SEARCHED (Int.Cl.7)**

A61K

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 28 May 2004 | Bayrak, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent
Office

INCOMPLETE SEARCH
SHEET C

Application Number

EP 03 02 4526

Although claims 2-24 are directed to a method of treatment of the human/animal body (Article 52(4) EPC), the search has been carried out and based on the alleged effects of the compound/composition.

-----

Claim(s) searched incompletely:
1-24 (all partially)

Claim(s) not searched:
-

Reason for the limitation of the search:

1. Present claims 1-24 relate to a compounds defined by reference to a desirable characteristic or property, namely "fragment thereof" or "stem cell and/or progenitor cells, multipotent adult progenitor cells, endothelial progenitor cells, side population cells, lineage negative stem cells". The claims cover all compounds having this characteristic or property, whereas the application provides support within the meaning of Article 84 EPC and/or disclosure within the meaning of Article 83 EPC for only a very limited number of such compounds. In the present case, the claims so lack support, and the application so lacks disclosure, that a meaningful search over the whole of the claimed scope is impossible. Independent of the above reasoning, the claims also lack clarity (Article 84 EPC). An attempt is made to define the product/compound/method/apparatus by reference to a result to be achieved. Again, this lack of clarity in the present case is such as to render a  meaningful search over the whole of the claimed scope impossible.

2. Claims 1-24 are related to the prevention or treatment of diseases which are not clearly defined, namely conditions related to "organ dysfunction caused by ischemia", or ".....ishemia is caused by...", "..to a patient who is subjected to a surgical or interventional procedure in order to improve organ function,..", "...ishemia causes organ defects", "wherein said surgical procedure is a procedure...", "G-CSF or fragment thereof is capable of recruiting stem and or /progenitor cells", "..said cell home to organs which harbour defects...", "...cells are capable of repairing and/or regenerating said organs". Due to the functional definition of the claimed subject-matter, the scope of protection of the claims 1-24 is obscure and not limited to the treatment of said specified conditions in the description and/or the claims but, by contrast, embraces an undefined number of other conditions allegedly capable of being improved or prevented by the administration of Hedgehog protein. Therefore, the claims 1-24 lack support (Art. 84 EPC) and the application lacks disclosure (Art. 83 EPC). Independent of the above reasoning the said expressions are vague and unclear and leave the reader in doubt as to the meaning of the technical feature to which they refer, thereby rendering the definition of the subject-matter of claims 1-24 unclear (Article 84 EPC).

Consequently, the search has been carried out for those parts of the claims which appear to be clear, supported and disclosed, namely those

| | | |
|---|---|---|
| **European Patent**<br>**Office** | **INCOMPLETE SEARCH**<br>**SHEET C** | **Application Number**<br>EP 03 02 4526 |

parts relating to the use of G-CSF for the treatment of diseases as in claim 7 and mobilisation of cells as in claims 19-21; and with due respect to the generalidea of the invention.

## EP 1 527 785 A1

This annex lists the patent family members relating to the patent documents cited in the above-mentioned European search report.
The members are as contained in the European Patent Office EDP file on
The European Patent Office is in no way liable for these particulars which are merely given for the purpose of information.

28-05-2004

| Patent document cited in search report | | Publication date | Patent family member(s) | | Publication date |
|---|---|---|---|---|---|
| EP 1327449 | A | 16-07-2003 | AU | 8622101 A | 26-03-2002 |
| | | | CA | 2421966 A1 | 11-03-2003 |
| | | | EP | 1327449 A1 | 16-07-2003 |
| | | | US | 2004019184 A1 | 29-01-2004 |
| | | | CN | 1466463 T | 07-01-2004 |
| | | | WO | 0222163 A1 | 21-03-2002 |
| WO 0194420 | A | 13-12-2001 | AU | 7533901 A | 17-12-2001 |
| | | | CA | 2412436 A1 | 13-12-2001 |
| | | | EP | 1290033 A1 | 12-03-2003 |
| | | | JP | 2004509847 T | 02-04-2004 |
| | | | WO | 0194420 A1 | 13-12-2001 |
| WO 02099081 | A | 12-12-2002 | WO | 02099081 A2 | 12-12-2002 |
| | | | US | 2002198150 A1 | 26-12-2002 |
| US 2003147862 | A1 | 07-08-2003 | AT | 257392 T | 15-01-2004 |
| | | | CA | 2304354 A1 | 15-04-1999 |
| | | | DE | 69821011 D1 | 12-02-2004 |
| | | | DK | 1019082 T3 | 10-05-2004 |
| | | | WO | 9917798 A1 | 15-04-1999 |
| | | | EP | 1019082 A1 | 19-07-2000 |
| | | | JP | 2001518517 T | 16-10-2001 |
| DE 10033219 | A | 24-01-2002 | DE | 10033219 A1 | 24-01-2002 |

EPO FORM P0459

For more details about this annex : see Official Journal of the European Patent Office, No. 12/82